# EUROPEAN PATENT SPECIFICATION

(11) **EP 1 900 333 B1**
(45) Date of publication and mention of the grant of the patent: **21.10.2009**
(21) Application number: 07022943.0
(22) Date of filing: 08.08.2001
(51) Int. Cl.: A61B 17/34

(54) **Molded trocar latch**
Geformter Trokarverschluss
Loquet de trocart moulé

(30) Priority: 08.08.2000 US 223811 P
(43) Date of publication of application: 19.03.2008
(62) Divisional of application: 01962001.2
(73) Proprietor: Tyco Healthcare Group LP, Norwalk, Connecticut 06856 (US)
(72) Inventor: Stellon, Gene, Southington CT 06489 (US); Racenet, David C., Litchfield CT 06759 (US); Stearns, Ralph A., Bozrah CT 06334 (US); Lehman, Adam, Wallingford CT 06492 (US)
(74) Representative: HOFFMANN EITLE

(56) References cited:
- EP-A- 0 617 924
- US-A- 5 374 252

## Description

### BACKGROUND

### 1. Technical Field

The present disclosure relates to a surgical trocar assembly and, in particular, to a trocar assembly having an integral latch mechanism which locks a protective shield of the trocar assembly in a fixed position and prevents retraction within the housing to the trocar assembly. Subject matter related to this application is disclosed in commonly assigned U.S. Patent Application Serial No. 09/526,837 filed March 16, 2000, entitled "Modular Trocar System and Methods of Assembly", and U.S. Patent No. 5,352,274 entitled "SAFETY TROCAR", filed December 7, 1994 by Green, now U.S. Patent No. 5,486,190.

### 2. Background of Related Art

Trocars are sharp pointed instruments used to puncture a body cavity. Generally, a trocar includes an obturator having a sharp obturator lip for penetrating the cavity wall and a protective sleeve in which the obturator is coaxially positioned. The trocar further includes a trocar sleeve or cannula. Once the body cavity has been punctured by the trocar, the obturator is typically removed from the cannula, thereby leaving the cannula in place extending into the body cavity.

Endoscopic or laparoscopic procedures are then performed with surgical instruments, e.g., laparoscopes, dissectors, graspers, staplers introduced through the cannula.

Commercially available safety trocars includes a spring loaded safety shield which is adapted to cover the obturator tip once the body cavity has been entered so as to provide an increased level of protection to internal structures from puncture or laceration. Other available safety trocars incorporate latching mechanisms having complex trigger and linking arrangement for locking and unlocking the protective sleeve. For example, a convenient location of the trigger on the trocar may require at least one intermediary linking mechanism to operatively connect the trigger with the locking member. The intermediary linking mechanism moves the locking member for the locking or unlocking of the shield in response to an operator's movement of the trigger. Latch mechanisms also generally require multiple bias members to ensure the positioning and the desired interrelation of the components such as the blocking member and linking mechanism. These configurations often require tightly controlled tolerances and are complex and time consuming to assemble. Additional complications include the manual adjusting of the interrelated components, such as the biased elements, after manufacturing in order to ensure the desired operational quality and reliability of the device.

In U.S. Patent No. 5,374,252, Banks et al. disclose a safety pneumoneedle through which the abdomen can be inflated with fluid such as insufflating gas. The pneumoneedle comprises a housing having front and rear ends, exterior surfaces adapted to be manually grasped, inner surfaces defining an elongate cavity having a fluid passageway portion. The pneumoneedle includes a non-removable cannula having a proximal end sealingly and fixedly attached to the housing, and a sharpened distal end adapted to pierce tissue. A protector assembly is present which has a closed blunt distal end, a first abutment end surface, and a camming surface. The protector assembly is movable relative to the cannula between a protecting position with the blunt distal end of the protector extending beyond the sharp distal end of the cannula to restrict engagement between tissue and the sharp distal end of the cannula, and a retracted position with portions of the sharp distal end of the cannula extending beyond the blunt distal end of the protector to afford puncture of tissue. The pneumoneedle also includes a latching and release mechanism for positively locking the protector in the protecting position after the cannula has pierced tissue and for releasing the protector after it has been locked in the protecting position.

However, a continuing need exists for a trocar latch mechanism that is monolithically molded as an assembly requiring a minimal number or even no additional components. A continuing need also exists for a trocar latch mechanism that reduces manufacturing costs by minimizing the need for manual post manufacturing adjusting of the latch mechanism.

### SUMMARY

A molded trocar latch apparatus is provided as defined in claim 1, the apparatus including a trocar housing and a shield. The housing includes a first monolithically formed portion or cap and a second monolithically formed portion or base. A latch mechanism is monolithically formed from one of the base and cap of the housing. The latch mechanism includes a slider and a latch. The slider is preferably movable between a first position and a second position by an operator. The slider and the latch are preferably operatively connected to move the latch between a locked and an unlocked position. The shield may include a cam positioned to disengage the slider from the latch. Upon disengagement, the latch is configured to return to the locked position and the first position.

The presently disclosed molded trocar latch, together with attendant advantages, will be best understood by reference to the following detailed description in conjunction with the figures below.

### Brief Description of the Drawings

Preferred embodiments of the present disclosure are described herein with reference to the drawings wherein:
FIG. 1 is a cross sectional side view of a trocar assembly incorporating the latch mechanism of the present disclosure, illustrating a molded latch mechanism utilizing a cross beam latch;
FIG. 2 is a cross sectional top view of the latch mechanism of FIG. 1 along lines B-B;
FIG. 3 is a cross sectional side view of FIG. 2 along lines C-C showing the slider;
FIG. 4 is a cross sectional side view of FIG. 2 along lines D-D;
FIG. 5 is a cross sectional perspective side view of the second embodiment of the molded trocar latch mechanism utilizing an elongate cantilevered beam molded trocar latch constructed in accordance with the present disclosure;
FIG. 6 is a second cross sectional side view of the elongate cantilevered beam molded trocar latch of FIG. 5;
FIG. 7 is a cross sectional side view of FIG. 6 along lines C-C;
FIG. 8 is a cross sectional side view of FIG. 7 along lines B-B; and
FIG. 9 is a simplified top view of the elongate cantilevered beam molded trocar latch mechanism showing the slider and the clearance cut around the slider.

### DETAILED DESCRIPTION

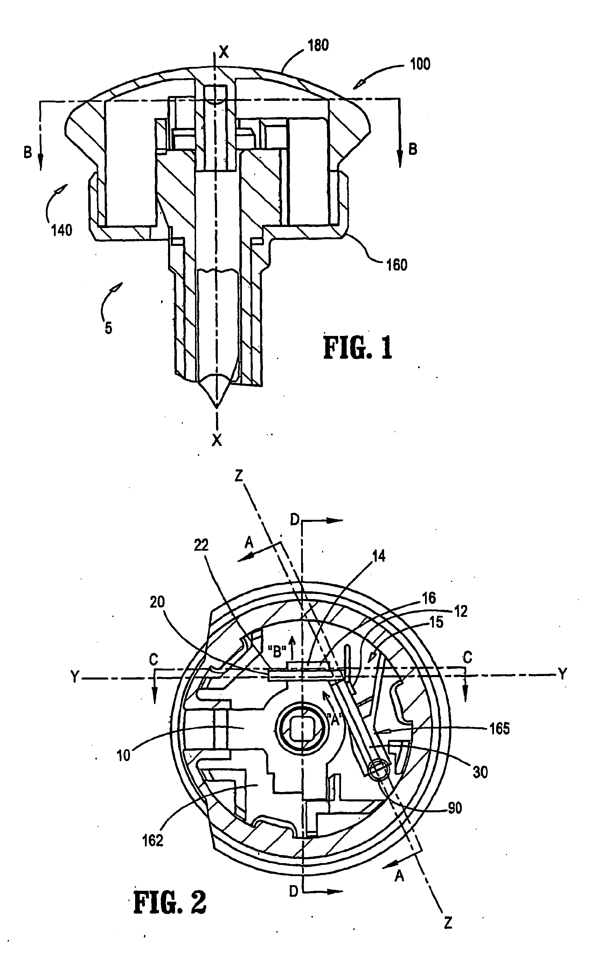

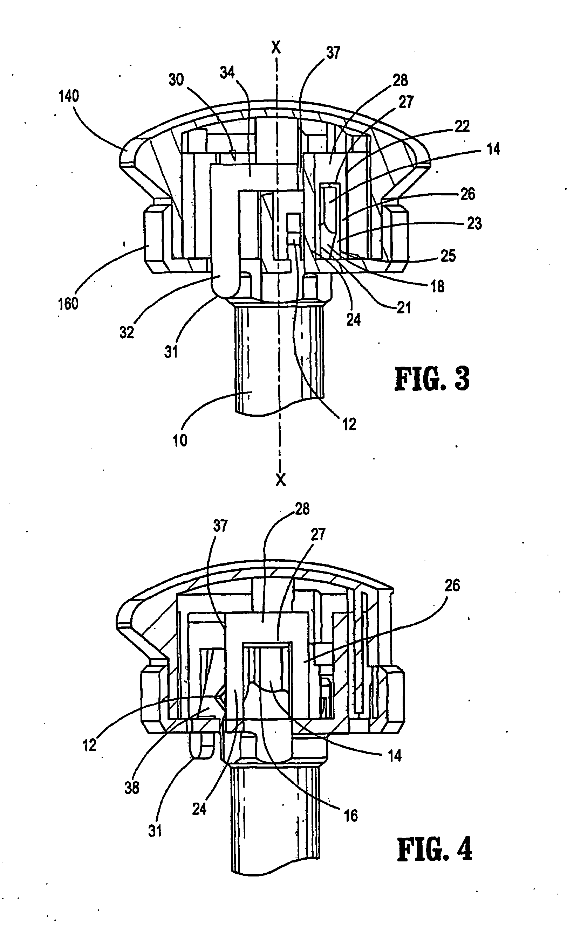

Referring initially to FIGS. 1-4, a trocar molded latch system 100 is configured for selectively positioning a latch mechanism 15 in a locked position and an unlocked position with a shield 10. In the locked position, shield 10 is physically positioned over a trocar blade or a sharpened obturator edge, for example, to prevent accidental lacerations. The unlocked position allows shield 10 to be moved proximally, against a bias, to expose the blade of the trocar during operational use such as, for example, during a percutaneous penetration of a patient. Trocar molded latch system 100 includes a housing 140, a base or a second portion 160, a cap or a first portion, shield 10, and latch mechanism 15. Base 160 and cap 180 are molded from a medical grade plastic as monolithic assemblies and then integrated into housing 140 of trocar latch system 100. Base 160 may be monolithically formed with the cannula of trocar assembly 5 or in the alternative, molded trocar latch 100 may be configured to connect with a cannula housing of trocar 5 as a removable assembly. Latch mechanism 15 includes a latch 20 and an actuating mechanism 30. Shield 10 is a movable assembly having a proximal end including a cam 12 and ledge 14. Trocar assembly 5 defines a central longitudinal axis-X. A bias 90, such as a compression spring, may be provided to assist shield 10 in backing off or moving distally from within housing 140. Bias 90 may be a separate assembly or formed as part of the base or cap or in combinations thereof.

In a first preferred embodiment, latch 20 is a cantilevered beam structure 22 monolithically formed with and extending proximally from a floor 162 of trocar base 160. Cantilevered beam 22 has two parallel beams or legs 24 and 26 that extend axially in a proximal direction, parallel with the longitudinal axis-X. A cross member 28, approximately perpendicular to the longitudinal axis is positioned on the proximal ends of legs 24 and 26. The locked position for cantilevered beam 22 is when legs 24 and 26 are approximately aligned with the central longitudinal axis.

Inside edges 21 and 23 of legs 24 and 26, respectively, a distal edge 27 of cross member 28, and floor 162 define a hole 25. Hole 25 is configured and dimensioned for receiving and engaging ledge 14 of shield 10 and thereby blocking further proximal movement of shield 10. Ledge 14 extends generally perpendicular to and radially from the surface of shield 10. Ledge 14 has a modified block shape with a flat planar proximal end 16 extending radially from the tubular surface of shield 10 and a distally tapered distal end 18. Legs 24 and 26 in combination with cross beam 28 define an axis-Y perpendicular to the central longitudinal axis-X. Cantilevered beam 22 is configured as a flexible structure biased to the locked or first position as a result of its materials of construction and structural shape.

A slider or actuating mechanism 30 is also monolithically formed with base 160 and is positioned at least partially in a slot 165 defined by floor 162. Slider 30, in this first preferred embodiment, has a generally right angled cantilevered beam configuration 32 having a beam or leg 34 aligned with the longitudinal axis-X and a cross beam 36 perpendicular to leg 34. The distal end of leg 34 has a rounded tip 31 that extends through slot 165 and is positioned at least partially distal to base 160. Slider 30 is connected to base 160 by a member 38 which is also configured as an area or point of flexure or pivoting. Leg 34 and cross beam 36 define an axis-Z perpendicular to longitudinal axis-X. Cross beam 36 has a tip 37 in apposition with cantilevered beam 22 when slider 30 is in the first position. Slider 30 is configured as a flexible structure biased to the locked or first position as a result of its materials of construction and structural shape and moveable between the first position and a second position wherein in the second position slider 30 has engaged latch 20 and repositioned latch 20 to the unlocked position.

Slider 30 is configured to pivot or flex along the direction of axis-Z against its inherent bias to intersect with cantilevered beam 22. Similarly, cantilevered beam 22 is configured to flex or pivot against its inherent bias about axis-Y. In this preferred embodiment, slider 30, moving along axis-Z, is configured to physically contact cantilevered beam 22, positioned along axis-Y, at a relative angle of approximately 115 degrees. The relative angle between the slider and latch can be varied in this embodiment through a range of approximately 75 degrees in any direction from a line perpendicular to the axis-Y and axis-X.

In operation, molded latch system 100 is in the locked position when shield ledge 14 is positioned in hole 25 and distal edge 27 of cantilevered beam 22 is engaging the proximal side 16 of shield ledge 14 precluding further proximal movement of shield 10. Molded latch system 100 is moved from the locked position to the unlocked position by an operator displacing actuating tip 31 in a generally proximal direction thereby pivoting slider 30 in the direction of axis-Z to directly engage tip 37 with cross beam 28. The displacement of tip 37 drives cross beam 28 to pivotally rotate cantilevered beam 22 about axis-Y and for cross beam 28 to be sufficiently displaced in the direction of arrow "A" to intersect with slider 30 such that latch 20 is driven radially outward in the direction of arrow "B" and out of contact with ledge 14, freeing shield 10 to move proximally and expose the blade of the trocar. This is the unlocked position of latch 100.

The proximal movement of shield 10 during operational use, such as during a percutaneous penetration, moves shield cam 12 from a first position at least partially distal to slider 30, to a second position proximal to slider 30. The translation of shield cam 12 between the first and second position contacts and repositions cross beam 36 of slider 30 in a radially direction away from shield 10 to displace slider 30 from being in apposition with cantilevered beam 22. When slider 30 is placed in the second position by an operator to reposition cantilevered beam 22 to the unlocked position, the contact of shield cam 12 with cross beam 36 disengages slider 30 from cantilevered beam 22. Cantilevered beam 22, as a result of its bias, then returns to the locked position and is prepared to engage ledge 14 and block further proximal movement of shield 10.

Alternatively, when slider 30 is released by the operator, the inherent bias in slider 30 will return it to the first position, removing the displacing force against cantilevered beam 22. Cantilevered beam 22 being similarly biased, then returns to the locked position Thus, once tip 31 is released, molded trocar latch 100 is automatically returned as a result of its bias to the locked position and is prepared to engage and block proximal movements of shield 10 that would expose the obturator blade.

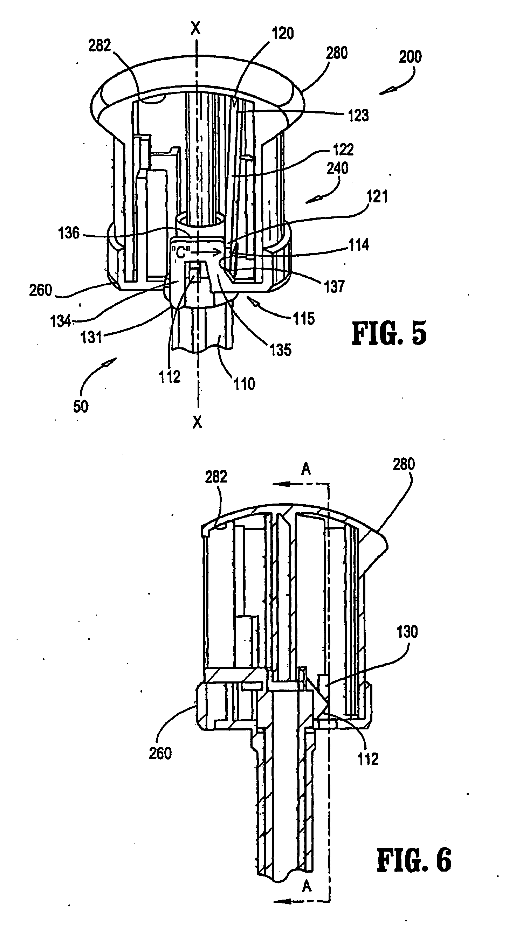

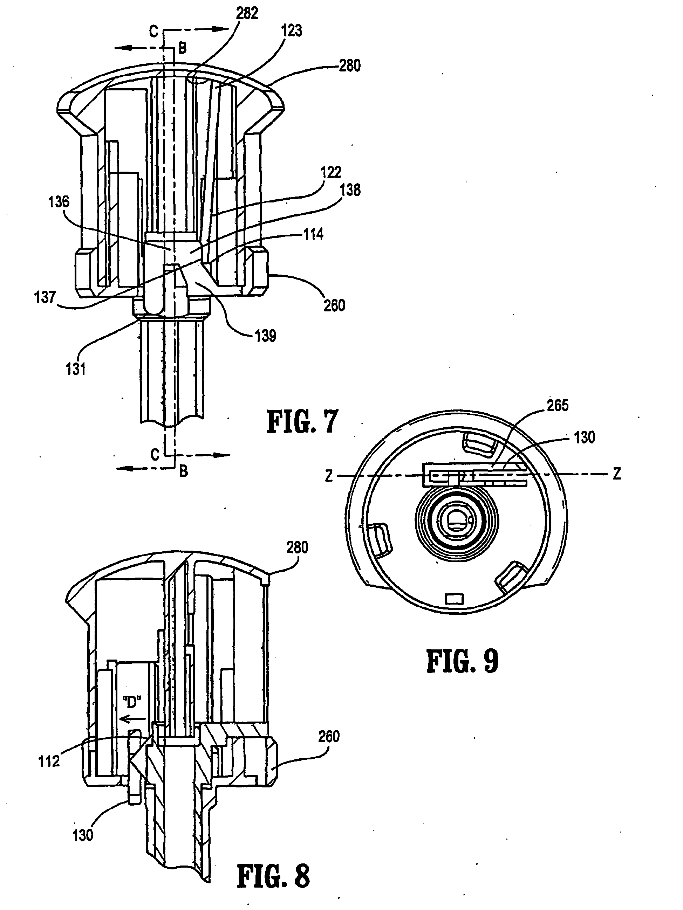

In FIGS. 5-9, a second embodiment of trocar molded latch system 200 includes a housing 240, base or second portion 260, cap or first portion 280, a shield 110, and a latch mechanism 115. Base 260 and cap 280 are molded from a medical grade plastic as monolithic assemblies and then integrated into housing 240 of trocar latch system 200. Latch mechanism 115 includes a latch 120 and an actuating mechanism 130. Shield 110 is a movable assembly having a proximal end including a cam 112 and ledge 114. Trocar assembly 50 defines a central longitudinal axis-X. A bias 190, such as a compression spring, may be provided to assist shield 110 in backing off or moving distally from within housing 240.

In the second preferred embodiment, latch 120 is an elongate cantilevered beam 122 defining an axis transverse to the longitudinal axis and monolithically formed with cap 280. Latch 120 includes a proximal end 123 connected to an interior wall 182 of cap 280 and extending in a distal direction to a distal end or tip 121. Tip 121 is positioned in apposition with shield 10 to block shield ledge 114 from any further proximal movement beyond tip 121. Tip 121 is in a locked position when it is directly contacting ledge 114 and shield 110 is blocked from retracting into housing 240. Cantilevered beam 122 is configured as a generally axially rigid structure in the direction of the longitudinal axis of beam 122, but has a controlled degree of flexibility in a direction perpendicular to the longitudinal axis of beam 122 to accommodate displacement from slider 30. Cantilevered beam 122 is biased to the locked position as a result of its materials of construction and structural shape.

Slider or actuating mechanism 130 is monolithically formed with base 260 and is positioned at least partially in a slot 265 defined by floor 262, and movable between a first position and a second position by an operator. Slider 130, in this first preferred embodiment, has a generally inverted "U" shaped cantilevered beam configuration 132 having a first beam 134 aligned with and a second beam 135 at least partially aligned with the longitudinal axis-X. Beams 134 and 135 are connected by a cross beam 136. The distal end of first leg 134 has a rounded tip 131 that extends through a slot 265 in base 260 and is positioned at least partially distal to base 260. Second beam 135 has a first portion 138 generally aligned with the longitudinal axis and a second portion 139 transverse to the longitudinal axis. Slider 130 is connected to base 260 through second beam 135 which is at least partially configured as an area or point of flexure or pivoting for slider 130. Leg 134, leg 135, and cross beam 136 define an axis-Z perpendicular to longitudinal axis-X. Cross beam 136 and portion 138 have an edge or and an inside corner between portion 139 positioned at least partially in apposition with cantilevered beam 122 when slider 130 is in the first position. Slider 130 is configured as a flexible structure biased to the locked position as a result of its materials of construction and structural shape.

Slider 130 is configured to pivot or flex along an axis-Z in the direction of arrow "C" against its inherent bias from the first position to intersect with and displace cantilevered beam 122. Similarly, cantilevered beam 122 is configured to flex or pivot against its inherent bias in the direction of arrow "C" to an unlocked position clear of ledge 114 when slider 130 is in the second position.

In operation, molded latch system 200 is in the locked position when proximal side 16 shield ledge 114 is positioned abutting tip 121 of cantilevered beam 122 precluding further proximal movement of shield 10. Molded latch system 200 is moved from the locked position to the unlocked position by an operator displacing actuating tip 131 in a generally proximal direction thereby pivoting slider 130 in the direction of axis-Z to directly engage edge 137 with beam 122 in the vicinity of tip 121. The displacement of tip 121 by edge 137 pivotally rotates or flexes cantilevered beam 122 about proximal end 123 sufficiently to displace tip 121 in the direction of arrow "C", generally aligned with axis-Z, to clear ledge 114, freeing shield 110 to move proximally and expose the trocar blade. This is the unlocked position of latch 200.

The continued proximal movement of shield 110 during operational use, such as during a percutaneous penetration, positions shield cam 112 proximal to slider 130. When shield cam 112 moves distally, it then intersect with cross beam 136 of slider 130 such that cross beam 136 is driven radially outward in the direction of arrow "D" and out of contact with cantilevered beam 122. Cantilevered beam 122, as a result of its bias, then returns to the locked position and is prepared to engage ledge 14 and block further proximal movement of shield 110.

Alternatively, when slider 130 is released by the operator, the inherent bias in slider 130 returns slider 130 to the first position, removing the displacing force against cantilevered beam 122. Cantilevered beam 122 being similarly biased, then returns to the locked position abutting shield 110. Thus, once tip 131 is released, molded trocar latch 200 is automatically returned as a result of its bias to the latched position and is prepared to engage and block proximal movements of shield 110 that would expose the obturator blade.

Although the illustrative embodiments of the present disclosure have been described herein with reference to the accompanying drawings, it is to be understood that the disclosure is not limited to those precise embodiments, and that various other changes and modifications may be affected therein by one skilled in the art without departing from the scope of the disclosure. For example, the molded geometry of individual components in the embodiments of the present disclosure, such as the slider and latch, can vary in origin from the base or the cap. Similarly, the geometry of the configuration of the ledge and cam of the shield depend upon on the specific design requirements of a given application of the molded trocar latch system. Further, the trocar housing or portions thereof may be configured as a removably and replaceable assembly with connections suitable for coupling with the trocar cannula.

It is thus understood that various modifications can be made to the various embodiments of the present invention without departing from its scope. Therefore the above description should not be construed as limiting the invention, but merely as presenting preferred embodiments of the invention. All such changes and modifications are intended to be included within the scope of the appended claims.

## Claims

1. A molded trocar latch apparatus and a shield comprising:
a trocar housing (140, 240) and a shield (10, 110), the trocar housing (140, 240) and the shield (10, 110) defining a central longitudinal axis (X), the housing (140, 240) including a base (160, 260) and a cap (180, 280); and
a latch mechanism (100, 200) configured for securing the shield (10, 110) in a locked position, the latch mechanism (100, 200) adapted to be actuated by an operator and directly operatively connected to the shield (10, 110), the latch mechanism (100, 200) being monolithically formed with at least one of the base (160, 260) and the cap (180, 280) of the trocar housing (140, 240) and configured for locking and unlocking the shield (10, 110),
the latch mechanism (100, 200) including a latch (20, 120) and a slider (30, 130), the latch (20, 120) and the slider (30, 130) being operatively connected with the shield (10, 110),
the latch (20, 120) being monolithically formed with the cap (180, 280),
**characterized in that**
the slider (30, 130) is monolithically formed with the base (160, 260).

2. The molded trocar latch of claim 1, wherein the shield has a fixedly positioned ledge (14, 114), the latch mechanism being configured to lock and unlock the ledge.

3. The molded trocar latch of claim 1, wherein the shield includes a shield cam (12, 112) fixedly positioned on the shield (10, 110), the shield being movable along the central longitudinal axis, the shield cam being operatively connected with the latch mechanism, the latch mechanism being positionable by an operator between a locked position and an unlocked position, the cam being configured to reposition the latch mechanism from the unlocked position to the locked position.

4. The molded trocar latch of claim 1, wherein the slider is configured to move between a first position and a second position and the latch is configured to move between a locked and an unlocked position, the slider being configured to be moved by an operator, the slider being operatively connected with the latch to move the latch between the locked and the unlocked position.

5. The molded trocar latch apparatus of claim 4, wherein the latch is biased to a locked position and the slider is biased to a first position.

6. The molded trocar latch of claim 5, wherein the slider is configured to move between a first position and a second position and the latch is configured to move between a locked and an unlocked position, the slider being configured to be moved by an operator, the slider being operatively connected with the latch to move the latch between the locked and the unlocked position.

7. The molded trocar latch of claim 6, wherein the latch has a cantilevered beam configuration, the cantilevered beam (22) defining a hole (25).

8. The molded trocar latch of claim 7, wherein the shield includes a ledge (14) and a locked position for the shield is defined by the positioning of the ledge in the hole in the cantilevered beam.

9. The molded trocar latch of claim 6, wherein the slider is a flexible cantilevered beam (32) having a right angled shape.

10. The molded trocar latch of claim 6, wherein the slider is configured to engage the latch at an angle within a range of approximately 75 degrees from a line perpendicular to the axis of rotation of the latch and the central longitudinal axis.

11. The molded trocar latch of claim 1, wherein the slider is configured to move between a first position and a second position and the latch is configured to move between a locked and an unlocked position, the slider being configured to be moved by an operator, the slider being operatively connected with the latch to move the latch between the locked and the unlocked position.

12. The molded trocar latch apparatus of claim 1, wherein the latch (120) is an elongate cantilevered beam (122) defining a longitudinal axis transverse to the central longitudinal axis and extending at least partially distally from the cap.

13. The molded trocar latch of claim 1, wherein the trocar housing includes a bias configured for backing off the shield.

14. The molded trocar latch of claim 12, wherein the latch has a tip (121) and the shield has a ledge (114), the tip being configured for abutting the ledge of the shield and stopping the proximal movement of the shield.

15. The molded trocar latch of claim 1, wherein the slider has an inverted "U" shaped cantilevered beam configuration.

16. The molded trocar latch of claim 1, wherein the latch includes a proximal end (123) and a distal end (121) defining a longitudinal axis (X), the latch being flexibly movable about the proximal end in a direction perpendicular to the longitudinal axis of the latch and generally rigid in a direction aligned with the longitudinal axis of the latch, the latch being movable between a locked and an unlocked position.

17. The molded trocar latch of claim 1, wherein the latch and the slider are configured as flexibly movable cantilevered beams.

## Patentansprüche

1. Geformte Trokarriegeleinrichtung und Abschirmung, umfassend:
ein Trokargehäuse (140, 240) und eine Abschirmung (10, 110), wobei das Trokargehäuse (140, 240) und die Abschirmung (10, 110) eine mittlere Längsachse (X) definieren, wobei das Gehäuse (140, 240) einen Boden (160, 260) und eine Kappe (180, 280) enthält; und
eine Riegelvorrichtung (100, 200), die dafür gestaltet ist, die Abschirmung (10, 110) in einer verriegelten Position zu befestigen, wobei die Riegelvorrichtung (100, 200) angepasst ist, um von einem Benutzer betätigt zu werden und direkt operativ mit der Abschirmung (10, 110) verbunden zu sein, wobei die Riegelvorrichtung (100, 200) monolithisch mit zumindest einem des Bodens (160, 260) und der Kappe (180, 280) des Trokargehäuses (140, 240) ausgebildet ist und zum Verriegeln und Entriegeln der Abschirmung (10, 110) gestaltet ist,
wobei die Riegelvorrichtung (100, 200) einen Riegel (20, 120) und einen Gleiter (30, 130) enthält, wobei der Riegel (20, 120) und der Gleiter (30, 130) operativ mit der Abschirmung (10, 110) verbunden sind,
wobei der Riegel (20, 120) monolithisch mit der Kappe (180, 280) ausgebildet ist,
**dadurch gekennzeichnet, dass** der Gleiter (30, 130) monolithisch mit dem Boden (160, 260) ausgebildet ist.

2. Geformter Trokarriegel nach Anspruch 1, wobei die Abschirmung eine fest positionierte Leiste (14, 114) aufweist, wobei die Riegelvorrichtung so gestaltet ist, dass sie die Leiste verriegelt und entriegelt.

3. Geformter Trokarriegel nach Anspruch 1, wobei die Abschirmung einen fest an der Abschirmung (10, 110) positionierten Abschirmungsnocken (12, 112) enthält, wobei die Abschirmung entlang der mittleren Längsachse beweglich ist und der Abschirmungsnocken operativ mit der Riegelvorrichtung in Verbindung steht, wobei die Riegelvorrichtung durch einen Benutzer zwischen einer verriegelten Position und einer entriegelten Position positionierbar ist, wobei der Nocken so gestaltet ist, dass die Riegelvorrichtung aus der entriegelten Position in die verriegelte Position zurückpositioniert wird.

4. Geformter Trokarriegel nach Anspruch 1, wobei der Gleiter gestaltet ist, um sich zwischen einer ersten Position und einer zweiten Position zu bewegen, und der Riegel gestaltet ist, um sich zwischen einer verriegelten und einer entriegelten Position zu bewegen, wobei der Gleiter gestaltet ist, um durch einen Benutzer bewegt zu werden,
wobei der Gleiter mit dem Riegel operativ verbunden ist, um den Riegel zwischen der verriegelten und der entriegelten Position zu bewegen.

5. Geformte Trokarriegeleinrichtung nach Anspruch 4, wobei der Riegel in eine verriegelte Position vorgespannt ist und der Gleiter in eine erste Position vorgespannt ist.

6. Geformter Trokarriegel nach Anspruch 5, wobei der Gleiter so gestaltet ist, dass er sich zwischen einer ersten Position und einer zweiten Position bewegt, und der Riegel so gestaltet ist, dass er sich zwischen einer verriegelten und einer entriegelten Position bewegt, wobei der Gleiter so gestaltet ist, dass er von einem Benutzer bewegt wird,
wobei der Gleiter operativ mit dem Riegel verbunden ist, um den Riegel zwischen der verriegelten und der entriegelten Position zu bewegen.

7. Geformter Trokarriegel nach Anspruch 6, wobei der Riegel eine Gestaltung mit frei tragendem Balken aufweist, wobei der frei tragende Balken (22) ein Loch (25) definiert.

8. Geformter Trokarriegel nach Anspruch 7, wobei die Abschirmung eine Leiste (14) enthält und eine verriegelte Position für die Abschirmung durch die Positionierung der Leiste in dem Loch (25) in dem frei tragenden Balken definiert ist.

9. Geformter Trokarriegel nach Anspruch 6, wobei der Gleiter ein flexibler frei tragender Balken (32) mit einer rechtwinkligen Form ist.

10. Geformter Trokarriegel nach Anspruch 6, wobei der Gleiter so gestaltet ist, dass er mit dem Riegel unter einem Winkel eingreift, der im Bereich von ungefähr 75° von einer Linie senkrecht zur Rotationsachse des Riegels und der mittleren Längsachse liegt.

11. Geformter Trokarriegel nach Anspruch 1, wobei der Gleiter so gestaltet ist, dass er sich zwischen einer ersten Position und einer zweiten Position bewegt, und der Riegel so gestaltet ist, dass er sich zwischen einer verriegelten und einer entriegelten Position bewegt, wobei der Gleiter so gestaltet ist, dass er von einem Benutzer bewegt wird,
wobei der Gleiter mit dem Riegel operativ verbunden ist, um den Riegel zwischen der verriegelten und der entriegelten Position zu bewegen.

12. Geformte Trokarriegeleinrichtung nach Anspruch 1, wobei der Riegel (120) ein länglicher frei tragender Balken (122) ist, der eine quer zu der mittleren Längsachse verlaufende und sich zumindest teilweise distal von der Kappe erstreckende Längsachse definiert.

13. Geformter Trokarriegel nach Anspruch 1, wobei das Trokargehäuse eine Vorspannung enthält, die dazu gestaltet ist, die Abschirmung zurück zu halten.

14. Geformter Trokarriegel nach Anspruch 12, wobei der Riegel eine Spitze (121) besitzt und die Abschirmung eine Leiste (114) besitzt, wobei die Spitze so gestaltet ist, dass sie an die Leiste der Abschirmung anschlägt und die proximale Bewegung der Abschirmung stoppt.

15. Geformter Trokarriegel nach Anspruch 1, wobei der Gleiter eine Gestaltung in Form eines umgekehrten "U"-förmigen frei tragenden Balkens aufweist.

16. Geformter Trokarriegel nach Anspruch 1, wobei der Riegel ein proximales Ende (123) und ein distales Ende (121) enthält, die eine Längsachse (X) definieren, wobei der Riegel um das proximale Ende in einer Richtung senkrecht zur Längsachse des Riegels flexibel beweglich ist und im Allgemeinen in einer Richtung, die mit der Längsachse des Riegels ausgerichtet ist, starr ist, wobei der Riegel zwischen einer verriegelten und einer entriegelten Position beweglich ist.

17. Geformter Trokarriegel nach Anspruch 1, wobei der Riegel und der Gleiter als flexibel bewegbare frei tragende Balken gestaltet sind.

## Revendications

1. Dispositif à verrou moulé pour trocart et un blindage comprenant:
un boîtier de trocart (140, 240) et un blindage (10, 100) le boîtier de trocart (140, 240) et le blindage (10, 110) définissant un axe longitudinal central (X), le boîtier (140, 240) comportant une base (160, 260) et un couvercle (180, 280); et
un mécanisme de verrouillage (100, 200) configuré pour fixer le blindage (10, 110) dans une position verrouillée, le mécanisme de verrouillage (100, 200) étant apte à être actionné par un opérateur et est relié fonctionnellement directement au blindage (10, 110), le mécanisme de verrouillage (100, 200) étant formé d'une manière monolithique avec au moins un parmi la base (160, 260) et le couvercle (180, 280) du boîtier de trocart (140, 240) et configuré pour verrouiller et déverrouiller le blindage (10, 110),
le mécanisme de verrouillage (100, 200) comportant un verrou (20, 120) et un coulisseau (30, 130), le verrou (20, 120) et le coulisseau (30, 130) étant fonctionnellement reliés au blindage (10, 110), le verrou (20, 120) étant formé de manière monolithique avec le couvercle (180, 280), **caractérisé en ce que** le coulisseau (30, 130) est réalisé monolithiquement avec la base (160, 260).

2. Verrou moulé pour trocart selon la revendication 1, dans lequel le blindage comporte une partie d'appui placée fixement (14, 114), le mécanisme de verrouillage étant configuré pour verrouiller et déverrouiller la partie d'appui.

3. Verrou moulé pour trocart selon la revendication 1, dans lequel le blindage comprend une came de blindage (12, 112) positionnée fixement sur le blindage (10, 110), le blindage étant mobile le long de l'axe longitudinal central, la came de blindage étant fonctionnellement reliée au mécanisme de verrouillage, le mécanisme de verrouillage pouvant être positionné par un opérateur entre une position verrouillée et une position déverrouillée, la came étant configurée pour repositionner le mécanisme de verrouillage de la position déverrouillée à la position verrouillée.

4. Verrou moulé pour trocart selon la revendication 1, dans lequel le coulisseau est configuré pour se déplacer entre une première position et une deuxième position, et le verrou est configuré pour se déplacer entre une position verrouillée et une position déverrouillée, le coulisseau étant configuré pour être déplacé par un opérateur, le coulisseau étant fonctionnellement relié au verrou pour déplacer le verrou entre la position verrouillée et la position déverrouillée.

5. Dispositif à verrou moulé pour trocart selon la revendication 4, où le verrou est sollicité vers une position verrouillée, et le coulisseau est sollicité vers une première position.

6. Verrou moulé pour trocart selon la revendication 5, dans lequel le coulisseau est configuré pour se déplacer entre une première position et une seconde position, et le verrou est configuré pour se déplacer entre une position verrouillée et une position déverrouillée, le coulisseau étant configuré pour être déplacé par un opérateur, le coulisseau étant fonctionnellement relié au verrou pour déplacer le verrou entre la position verrouillée et la position déverrouillée.

7. Verrou moulé pour trocart selon la revendication 6, dans lequel le verrou a une configuration d'arbre en porte-à-faux, l'arbre en porte-à-faux (22) définissant un trou (25).

8. Verrou moulé pour trocart selon la revendication 7, dans lequel le blindage comporte une partie d'appui (14), et la position verrouillée pour le blindage est définie par le positionnement de la partie d'appui dans le trou pratiqué dans l'arbre en porte-à-faux.

9. Verrou moulé pour trocart selon la revendication 6, dans lequel le coulisseau est un arbre souple en porte-à-faux (32) ayant une forme en angle droit.

10. Verrou moulé pour trocart selon la revendication 6, dans lequel le coulisseau est configuré pour entrer en prise avec le verrou à un angle compris dans une plage de 75 degrés environ à partir d'une ligne perpendiculaire à l'axe de rotation du verrou et à l'axe longitudinal central.

11. Verrou moulé pour trocart selon la revendication 1, dans lequel le coulisseau est configuré pour se déplacer entre une première position et une seconde position, et le verrou est configuré pour se déplacer entre une position verrouillée et une position déverrouillée, le coulisseau étant configuré pour être déplacé par un opérateur, le coulisseau étant fonctionnellement relié au verrou pour déplacer le verrou entre la position verrouillée et la position déverrouillée.

12. Dispositif à verrou moulé pour trocart selon la revendication 1, dans lequel le verrou (120) est un arbre oblong en porte-à-faux (122) définissant un axe longitudinal transversal à l'axe longitudinal central et s'étendant au moins partiellement distalement du couvercle.

13. Verrou moulé pour trocart selon la revendication 1, dans lequel le boîtier de trocart comprend un biais configuré pour repousser le blindage.

14. Verrou moulé pour trocart selon la revendication 12, dans lequel le verrou possède une pointe (121), et le blindage possède une partie d'appui (114), la pointe étant configurée pour buter contre la partie d'appui du blindage et pour arrêter le mouvement proximal du blindage.

15. Verrou moulé pour trocart selon la revendication 1, dans lequel le coulisseau a une configuration d'arbre en porte-à-faux d'une forme de "U" inversé.

16. Verrou moulé pour trocart selon la revendication 1, où le verrou comprend une extrémité proximale (123) et une extrémité distale (121) définissant un axe longitudinal (X), le verrou étant déplaçable d'une manière flexible autour de l'extrémité proximale dans une direction perpendiculaire à l'axe longitudinal du verrou et généralement rigide dans une direction alignée avec l'axe longitudinal du verrou, le verrou étant déplaçable entre une position verrouillée et une position déverrouillée.

17. Verrou moulé pour trocart selon la revendication 1, où le verrou et le coulisseau sont configurés comme des arbres en porte-à-faux mobiles de manière flexible.
